# EUROPEAN PATENT SPECIFICATION

(11) **EP 0 556 158 B1**
(45) Date of publication and mention of the grant of the patent: **29.05.1996**
(21) Application number: 93810072.4
(22) Date of filing: 05.02.1993
(51) Int. Cl.: A61K 9/70

(54) **Process for preparing ultrasonically sealed transdermal drug delivery systems**
Verfahren zur Herstellung von ultraschallversiegelten transdermalen Arzneimittelabgabesystemen
Procédé pour la préparation de systèmes pour la libération de médicaments à usage transdermal scelles à l'aide l'utrasons

(30) Priority: 14.02.1992 US 837456
(43) Date of publication of application: 18.08.1993
(73) Proprietor: CIBA-GEIGY AG, CH-4002 Basel (CH)
(72) Inventor: Rudella, Michael D., Cedar Knolls, NJ 07666 (US)

(56) References cited:
- DATABASE WPI Week 7243, Derwent Publications Ltd., London, GB; AN 72-69291T

## Description

The present invention relates to a process for preparing transdermal drug delivery systems. More particularly, it relates to the method of sealing various laminar components of transdermal drug delivery systems to each other.

Transdermal drug delivery systems (TTS) of different types are known. In their simplest forms there are the matrix monolith type systems and the membrane "sack" type systems. The monolith type has an active agent which is contained within a solid or semi-solid (capable of retaining its shape under light to moderately applied pressure) which is cast on or adhered to a backing layer impermeable to the passage of active agent. An adhesive may be only on or around the perimeter of or over the entire exposed surface (oposite that adjacent the backing layer) of the active agent containing layer. The "sack" type system has an active agent impermeable layer and a second layer (active agent permeable) affixed thereto such that the two layers define an active agent bulk reservoir for containing an active agent formulation.

In the "sack" type transdermal drug delivery systems there are at least two components which must be sealed together, usually with the active agent or active agent formulation in place during the sealing operation. In the monolith type of system, the monolithic material has no adhesive properties at all or its adhesive properties are insufficient for the quality standards of commercial products. Therefore, the active agent containing material must be affixed to the backing layer by alternative means, such as sealing, or adhering it via an adhesive, or overlayering it with another layer which overlayer is affixed to the backing.

In the production of the above mentioned TTS, heat sealing and pressure sealing techniques have been used, either alone or in various combinations. While these techniques have been found acceptable for obtaining a product which will meet regulatory approval and quality assurance requirements, there are significant drawbacks involved that, if overcome, would improve the manufacture of such systems and the systems themselves more reliable.

The range of adhesives that will assure suitable integrity of seal areas and which will not affect active agent formulation components or their delivery from the reservoir is limited. Adhesives useful for seal purposes must be impermeable to active agent formulation so as to retain the active agent formulation within the system and not create a reservoir to which the active agent will preferentially migrate during storage. Such migration results in delivery characteristics differing from those for which the product was designed or results in an undesirable batch-to-batch variation.

Active agent impermeable adhesives often are unsuitable for sealing methods. In the event that the adhesive is in contact with the portion of the system through which the active agent is intended to diffuse, the delivery characteristics of the system will vary from the intended performance characteristics.

Pressure sealing eliminates the problems inherent in the chemical sealing, since there is no concern for chemical interaction between the sealing means and the active agent formulation and there is no concern for inappropriate migration of active agent into the sealing means. However, pressure seals have their own problems associated with them. In the process of applying the pressure seal the active agent formulation can easily be displaced and the materials being sealed can shear. Furthermore, in an effort to avoid inadvertent displacement of the active agent formulation, the seal might be placed at a slightly further point from the center, resulting in a space in which the active agent formulation might migrate within the reservoir area. This could result in differences in performance of the system depending upon orientation of the device during storage and use.

The alternative sealing technique, heat sealing, also overcomes the problems associated with adhesive sealing, but introduces other problem areas. In the heat sealing operation (or thermal-die process) a steel sealing die of specified geometry is heated to a temperature range significantly greater than the melting point of the thermoplastic materials being sealed together. High die temperatures are necessary to insure heat penetration through the thermoplastic materials and induce melt at the seal area. The problems inherent in this process are that excessive heat causes the laminate materials to shrink and distort. Machine speed and temperature of the die are proportionally related so that high die temperatures are needed for high speed operation, the laminate material with the lowest melting point may flow out of the seal area before the seal can be formed so that the thermal bond is weakened. The melted thermoplastic material remains in a hydraulic state for a brief period after the hot die is retracted during which tension forces may cause the laminate material to separate thereby weakening the seal. Finally, heat seal systems are limited to those components (including active agent formulation) which can withstand the temperatures applied without appreciable degradation.

In JP-A-47020327 sealing of laminates containing medicaments is described. The seals are formed by thermal sealing of the laminates and applying ultrasonic radiation
It is therefore an object of the invention to provide a process for sealing transdermal drug delivery systems which avoids the above problems of the known sealing techniques.

It is another object of the invention to provide a transdermal drug delivery system sealing means which reduces the percentage of reject systems in commercial production over those prepared using the former techniques set out above.

It is still another object of the invention to provide a transdermal drug delivery system sealing means which allow high machine speed sealing of such systems without excessive heating.

These and other objects are surprisingly achieved by a method of sealing components of a transdermal drug delivery system to one another utilizing ultrasonic welding.

The invention relates to a process for preparing a transdermal drug delivery system where the system has a backing layer and a second layer, said second layer contacting said backing layer and adhered thereto with a seal, said system further comprising an active agent located within said second layer or within a reservoir region defined by said backing layer and said second layer, wherein said backing layer is impermeable to said active agent and said second layer is permeable to said active agent or an activated form of said active agent. Each of the layers may be a single layer or a multilaminate. The second layer may be formed as a membrane and unless the second layer has already adhesive properties, an additional adhesive layer is needed, either in a peripheral region or over a portion of surface of the second layer distal to the backing, or adhesive in both such areas. A release liner covers the exposed portion of the second layer until removed during the process of applying the transdermal device to a patient. The invention, therefore, is related to the preparation of a transdermal drug delivery system which needs a seal by forming the seal ultrasonically.

Virtually any transdermal drug delivery device that needs a seal between ultrasonically sealable components can be used in the invention.

Ultrasonically sealable materials useful for the various components being sealed by the invention method include thermoplastic films. Preferably, these thermoplastics are either amorphous or crystalline. Highly preferred crystalline films for use in the invention include, without limitation, ethylene-vinyl acetate, fluoropolymers, polyamides, polybutylene, polyester, polypropylene, polyethylene, poly(vinyl acetate) and copolymers of mixtures of the monomers therein. Suitable copolymers include polymers having two or more different monomers selected from the monomers of the polymers in the previous sentence. Highly preferred amorphous materials include acrylic cellulose, polystyrene, poly(vinyl chloride), poly(vinyl dichloride), and copolymers containing at least two different monomers selected from the monomers of the above-mentioned amorphous polymers. Most highly preferred materials include ethylene-vinyl acetate, polyester, and polyethylene.

When either the backing layer or the second layer is itself a multilaminate, only the surfaces thereof which contact the other layer must consist of ultrasonically sealable material useful in the present invention. However, if desired, any of the other components of the multilaminates may be ultrasonically sealable.

According to the present invention, any layer or material described generally may be a single material or a composite laminate wherein at least one component of the laminate is the material described above and is still in a position to accomplish the results intended. For example, wherever an ultrasonically sealable material is indicated, that material may be a single material or a laminate thereof with other materials, provided the portion of such ultrasonically sealable material which is intended to be ultrasonically sealed is available for such sealing. Where a laminate contains more than one ultrasonically sealable material therein, only one of such ultrasonically available materials need be available for forming the seal according to the invention.

Active agents and active agent precursors may be selected from any compound which is transdermally administrable and systemically active per se or metabolized in vivo to an active agent. The active agent and active agent precursors may also be selected from dermally active agents and their precursors. These materials are, without limitation, preferably selected from: antitubercular agents, such as isoniazid and rifampin; analgesics such as fentanyl and sufentanyl; muscle relaxants, such as baclofen; β-adrenergic receptor agonists and antiasthmatics, such as theophylline, formoterol, and terbutaline; steroids, such as estradiol, progesterone, norethisterone acetate, methyltestosterone, and desoxycorticosterone; anticholinergics, such as scopolamine and methscopolamine; vasodilators, such as nitroglycerine; antihypertensives, such as metoprolol; antihistamines, such as triprolidine, tripelenamine, and diphenhydramine; cholinergic agents, such as arecoline; CNS stimulants, such as methylphenidate and nikethamide; angiotensin converting enzyme inhibitors such as benazepril, and benazeprilat; nicotine, physostigmine, and naloxone.

A preferred class of drugs for use in the systems prepared by the present invention method is fentanyl, sufentanyl, terbutaline, formoterol, theophylline, estradiol, norethisterone acetate, progesterone, scopolamine, nitroglycerine, triprolidine, tripelenamine, diphenhydramine, arecoline, nicotine, benazepril, and benazeprilat.

In the process for the preparation of the transdermal systems according to the present invention, the ultasonically sealable components are brought in contact and an ultrasonic radiation is applied so as to cause sealing in the contact region. The frequency used is in the range of 18 kHz to 22 kHz, preferably 19 kHz to 21 kHz, more preferably 20 kHz. The ultrasonic radiation can be applied for varying lengths of time, and, without limitation, is preferably applied for 350 msec to 950 msec, more preferably for 450 msec to 750 msec. In the present invention, pressure is also applied in the seal area during a substantial portion of the time the ultrasonic radiation is applied. This force is, without limitation, preferably 90 kg to 300 kg more preferably 150 kg to 250 kg most preferably 175 to 225 kg. Lesser pressures are also acceptable, if desired. The ultrasonic radiation is produced from so-called ultrasonic welding devices such as the ones marketed by Branson Corp., especially any type from the 900 series.

All other components of the transdermal systems of the invention may be chosen as appropriate from the published literature on transdermal drug delivery systems. Since the ultrasonic sealing technique is amenable to precise control, it is also suitable for forming preferentially burstable seals present in transdermal drug delivery systems. Preferentially weaker seals intended for bursting can be made by separately controlling the ultrasonic forces applied to specific areas of the seal regions. Utilizing shorter ultrasonic frequency application times, and less intense pressures than for the non-burstable seals will result in preferentially burstable seals as desired. Where pressure is not used to seal the system, the ultrasonic sealing frequency application time should not be greater than 90% of the value used for the main seals, preferably not greater than 75%, and most preferably not greater than 60%, to create the burstable seals. Where pressure is simultaneously used with the ultrasonics to seal the main seals, the burstable seals can be created using pressures and ultrasonic sealing frequency application times, the product of which is not greater than 90%, preferably not greater than 75%, most preferably not greater than 60%, of the product of the pressure and ultrasonic frequency application time used for the main seals.

Seals produced by ultrasonic radiation are notably different from seals produced by hot-die in the disposition of the thermoplastic material that is ejected from the seal area during the sealing process and its subsequent solidification. During ultrasonic sealing, vibration instantly melts the thermoplastic material that is in direct contact with the sealing tools. The ultrasonic vibration forces some of the molten material to vacate the contact area and solidify along the interface of the subject films, effectively fortifying the seal against avenues of leakage. Durin the ultrasonic sealing cycle, crystalline thermoplastic materials instantly melt upon the introduction of ultrasonic energy and solidify at the instant that the ultrasonic energy is removed. In the case of the presence of amorphous materials, ultrasonics are cycled and turned off while pressure is maintained, enabling the molten material to solidify before the removal of clamping pressure. In either case, the result is a hermetic seal, reinforced by a continuous bead of plastic along the interface of the subject materials.

### Examples:

The following Examples are for illustrative purposes only and do not limit the scope of the claimed invention.

### Example 1

An ultrasonically sealable laminate (impermeable to nitroglycerine), available under the tradename Scotchpack from 3M Corp., corresponding to the first backing layer, comprising a pigmented polyethylene, a polyester, vapor coated aluminum oxide, ethylene-vinyl acetate, is dispensed from lower mill roll unwinder and introduced into a transdermal fabrication machine. An ultrasonically sealable laminate, comprising an ethylene-vinyl acetate copolymer film, corresponding to the second layer, a silicon adhesive, and a polyester release liner is dispensed from upper mill roll unwinder and introduced into the transdermal drug delivery fabrication machine.

As both laminates are pulled through the fabrication machine, a nitroglycerine containing formulation is dispensed by a dispensing unit onto the ultrasonically sealable surface of the backing layer laminate. After deposition of the nitroglycerine formulation, the ultrasonically sealable surface of the second laminate is brought in contact with the ultrasonically sealable surface of the first laminate with the nitroglycerine formulation occupying a portion thereof. The loosely fitted system is then brought to an ultrasonic sealing unit, where the nitroglycerine formulation deposition area is precisely aligned with predetermined seal patterns on a male rotary sealing die located below the first laminate. The horn, located above the second laminate and aligned with the die, and the die approach each other so as to apply a force of 180 kg while from the horn an ultrasonic force of 20 kHz is generated to effect sealing. After sealing, the sealed systems move to the die cutting station where the finished transdermal systems are cut from the films by rotary die cutters and the waste is removed by take up roller.

### Example 2

A ultrasonically sealed transdermal delivery sytem is produced in a manner analogous to Example 1. Estradiol is chosen as active agent. The backing layer is a laminate comprising a polyester and ethylene-vinyl acetate.

## Claims

1. A process for preparing a transdermal drug delivery system said system comprising a backing layer and a second layer, said second layer contacting said backing layer and adhered thereto with a seal, said device further comprising an active agent located within said second layer or within a region defined by said backing layer and said second layer, wherein said backing layer is impermeable to said active agent and said second layer is permeable to said active agent or an activated form of said active agent, characterized in that the seals are formed by applying ultrasonic radiation and a pressure in the seal area.

2. The process according to claim 1, characterized in that an ultrasonic radiation of 18 kHz to 22 kHz is applied.

3. The process according to claim 1, characterized in that an ultrasonic radiation of 19 kHz to 21 kHz is applied.

4. The process according to claim 1, characterized in that an ultrasonic radiation of 20 kHz is applied.

5. The process according to claim 1, characterized in that the ultrasonic radiation is applied for 250 msec to 950 msec.

6. The process according to claim 1, characterized in that the ultrasonic radiation is applied for 450 msec to 750 msec.

7. The process according to claim 1, characterized in that the ultrasoninc radiation is applied substantially simultaneously with a force of 90 kg to 300 kg in the seal area.

8. The process according to claim 1, characterized in that the ultrasoninc radiation is applied substantially simultaneously with a force of 150 kg to 250 kg in the seal area.

## Patentansprüche

1. Verfahren zur Herstellung eines transdermalen Arzneimittelabgabesystems, wobei das System eine Stützschicht und eine zweite Schicht umfaßt, wobei die zweite Schicht in Kontakt mit der Stützschicht ist und daran mit einem Siegel haftet, wobei die Vorrichtung weiterhin einen innerhalb der zweiten Schicht oder innerhalb einer durch die Stützschicht und die zweite Schicht definierten Region lokalisierten Wirkstoff umfaßt, wobei die Stützschicht für den Wirkstoff impermeabel ist, und die zweite Schicht für den Wirkstoff oder eine aktivierte Form des Wirkstoffs permeabel ist, dadurch **gekennzeichnet,** daß die Siegel durch Aufbringen einer Ultraschallbestrahlung und eines Drucks auf die Siegelfläche gebildet werden.

2. Verfahren nach Anspruch 1, dadurch **gekennzeichnet**, daß eine Ultraschallbestrahlung von 18 kHz bis 22 kHz angewendet wird.

3. Verfahren nach Anspruch 1, dadurch **gekennzeichnet**, daß eine Ultraschallbestrahlung von 19 kHz bis 21 kHz angewendet wird.

4. Verfahren nach Anspruch 1, dadurch **gekennzeichnet**, daß eine Ultraschallbestrahlung von 20 kHz angewendet wird.

5. Verfahren nach Anspruch 1, dadurch **gekennzeichnet*****,*** daß die Ultraschallbestrahlung für 250 msec bis 950 msec angewendet wird.

6. Verfahren nach Anspruch 1, dadurch **gekennzeichnet**, daß die Ultraschallbestrahlung für 450 msec bis 750 msec angewendet wird.

7. Verfahren nach Anspruch 1, dadurch **gekennzeichnet**, daß die Ultraschallbestrahlung im wesentlichen gleichzeitig mit einer Kraft von 90 kg bis 300 kg auf der Siegelfläche angewendet wird.

8. Verfahren nach Anspruch 1, dadurch **gekennzeichnet**, daß die Ultraschallbestrahlung im wesentlichen gleichzeitig mit einer Kraft von 150 kg bis 250 kg auf der Siegelfläche angewendet wird.

## Revendications

1. Un procédé pour la préparation d'un système de libération transdermique de médicament, ce système comportant une couche support et une seconde couche, cette seconde couche venant en contact avec ladite couche support et y adhérant au moyen d'un joint d'étanchéité, ce dispositif comportant en outre un agent actif situé à l'intérieur de ladite seconde couche ou l'intérieur d'une région délimitée par ladite couche support et ladite seconde couche, cette couche support étant imperméable audit agent actif tandis que la seconde couche est perméable audit agent actif ou à une forme activée de cet agent actif, caractérisé en ce que les joints d'étanchéité sont formés par application d'une radiation ultrasonique et d'une pression sur la zone de soudure.

2. Le procédé selon la revendication 1, caractérisé en ce qu'une radiation ultrasonique de 18 kHz à 22 kHz est appliquée.

3. Le procédé selon la revendication 1, caractérisé en ce qu'une radiation ultrasonique de 19 kHz à 21 kHz est appliquée.

4. Le procédé selon la revendication 1, caractérisé en ce qu'une radiation ultrasonique de 20 kHz est appliquée.

5. Le procédé selon la revendication 1, caractérisé en ce que la radiation ultrasonique est appliquée pendant de 250 millisecondes à 950 millisecondes.

6. Le procédé selon la revendication 1, caractérisé en ce que la radiation ultrasonique est appliquée pendant de 450 millisecondes à 750 millisecondes

7. Le procédé selon la revendication 1, caractérisé en ce que la radiation ultrasonique est appliquée pratiquement de façon simultanée avec une force de 90 kg à 300 kg dans la zone de soudure.

8. Le procédé selon la revendication 1, caractérisé en ce que la radiation ultrasonique est appliquée de façon pratiquement simultanée avec une force de 150 à 250 kg dans la zone de soudure.
